Europäisches Patentamt

**European Patent Office**

**Office européen des brevets**

(19)

(11) Numéro de publication: **0 120 829**
**A2**

(12) # DEMANDE DE BREVET EUROPEEN

(21) Numéro de dépôt: **84870005.0**

(22) Date de dépôt: **06.01.84**

(51) Int. Cl.³: **C 12 N 15/00**

(30) Priorité: **21.02.83 BE 210157**

(43) Date de publication de la demande:
**03.10.84 Bulletin 84/40**

(84) Etats contractants désignés:
**AT BE CH DE FR GB IT LI LU NL SE**

(71) Demandeur: **Région Wallonne représentée par l'Exécutif Régional Wallon**
**11, Boulevard de l'Empereur**
**B-1000 Bruxelles(BE)**

(72) Inventeur: **Bollen, Alex**
**Gaasbeekstraat, 65**
**B-1711 Itterbek(BE)**

(72) Inventeur: **Herzog, Albert Alfred Maurice**
**Elewijtsesteenweg, 190**
**B-1840 Eppegem(BE)**

(72) Inventeur: **Chuchana, Paul**
**Chaussée de Vleurgat, 306**
**B-1050 Bruxelles(BE)**

(72) Inventeur: **Morais Gravador, Alfredo Jaime**
**Avenue des Cygnes, 21**
**B-1640 Rhode-St-Genese(BE)**

(72) Inventeur: **Herion, Pascal**
**Rue de Falisolle, 65**
**B-5700 Sambreville(BE)**

(74) Mandataire: **Blanchart, André**
**MINISTERE DE LA REGION WALLONNE Direction de l'Energie et des Technologies Nouvelles Place Joséphine Charlotte 3 (Boites 27-28)**
**B-5100 Jambes Namur(BE)**

(54) Procédé de préparation d'un clone bactérien produisant de l'alpha 1-antitrypsine humaine.

(57) Procédé de préparation d'un clone bactérien produisant l'α1-antitrypsine humaine par la formation de molécules hybrides à partir d'ADN complémentaire double brin obtenu d'ARN messagers totaux de foie humain, enrichis en molécules codant pour l'α1-antitrypsine humaine, avec un vecteur plasmide, par la transformation de bactéries hôtes Eschérichia Coli par des molécules hybrides, par la sélection des clones porteurs de l'ADN-AT caractéristique de l'α1-antitrypsine humaine et par l'identification parmi ces clones de ceux qui expriment l'α1-antitrypsine humaine.

EP 0 120 829 A2

Procédé de préparation d'un clone bactérien produisant de l'α 1-antitrypsine humaine.

La présente invention se rapporte à la préparation d'un clone bactérien produisant l'α 1-antitrypsine, humaine. Elle comprend plus spécialement un procédé de production d'un ADN (acide désoxyribonucléique) complémentaire double brin correspondant à l'information génétique caractéristique de l'α 1-antitrypsine humaine (cette molécule sera appelée ci-après ADN-AT). Elle est aussi relative à des vecteurs comportant l'ADN-AT, en vue de la mise en oeuvre de ses propriétés biologiques.

L'α 1-antitrypsine, l'un des composants majeurs du plasma sanguin, est synthétisée dans le foie et secrétée dans le plasma où sa demi-vie est de six jours. Cette enzyme fonctionne essentiellement en tant qu'inhibiteur d'enzymes protéolytiques. La cible principale de l'α 1-antitrypsine est l'élastase, une protéine impliquée dans la perte d'élasticité des tissus pulmonaires. L'α 1-antitrypsine, cependant, possède aussi une affinité certaine pour la plasmine et la thrombine; c'est là que se situe son rôle dans le processus de fibrinolyse. Par ailleurs, la déficience héréditaire en cette enzyme, phénomène assez fréquent, s'accompagne de troubles de dégénérescence précoce du poumon et, dans certains cas, de troubles hépatiques.

Il semble que des facteurs génétiques et de l'environnement participent ensemble à l'apparition des problèmes de santé. En effet, l'α 1-antitrypsine est un facteur de protection dont la déficience génétique rend l'individu sensible à des dégâts pulmonaires cumulatifs, suite aux processus inflammatoires induits par des agents polluants. Les enzymes protéolytiques relâchés dans l'organisme pendant les phases inflammatoires saturent la quantité limitée d'α 1-antitrypsine présente chez les individus déficients.

La situation est d'autant plus grave que l'$\alpha$1-antitrypsine peut être elle-même inactivée directement par certains agents polluants.

Si, pour des raisons génétiques ou autres, le taux d'$\alpha$1-antitrypsine dans le plasma tombe sous un certain seuil, des troubles pulmonaires graves se déclarent.

La perte d'élasticité des poumons conduit à des dégâts irréversibles, caractéristiques de l'emphysème.

La compréhension de la pathogénèse des troubles pulmonaires fournit la base de nouveaux concepts thérapeutiques comme, par exemple, la thérapie par remplacement d'anti-protéase. Une telle voie suppose notamment l'obtention de grandes quantités d'$\alpha$1-antitrypsine que l'on pourrait administrer aux emphysémateux par voie veineuse, ou la synthèse d'analogues de l'$\alpha$1-antitrypsine ayant une affinité accrue pour l'élastase et une sensibilité moindre à l'inactivation directe par les agents polluants.

La présente invention a pour objet un procédé de préparation d'un clone bactérien produisant l'$\alpha$1-antitrypsine humaine réalisé par le clonage dansune bactérie hôte d'un ADN complémentaire double brin à partir des ARN messagers de foie humain.

Ce procédé faisant l'objet de la présente invention permet d'obtenir l'$\alpha$1-antitrypsine humaine en quantités suffisantes, notamment pour l'utiliser dans des applications thérapeutiques.

Le procédé de préparation d'un clone bactérien produisant l'$\alpha$1-antitrypsine humaine selon la présente invention comprend la combinaison des étapes suivantes :

a) - l'extraction et la purification des ARN messagers totaux de foie humain;

b) - l'enrichissement de ces messagers en molécules codant pour l'$\alpha$1-antitrypsine humaine;

c) - la synthèse in vitro de l'ADN complémentaire double brin au départ de cette fraction messagère enrichie;

d) - l'enrichissement de la préparation d'ADN complémentaire double brin en molécules de taille correspondant à celle de l'ADN-AT;

e) - l'addition d'extrémités cohésives à cet ADN-AT;

f) - le mélange in vitro de cette fraction enrichie portant des extrémités cohésives avec un vecteur plasmide portant des extrémités cohésives complémentaires pour obtenir des molécules hybrides;

g) - la transformation des bactéries hôtes Escherichia Coli par ces molécules hybrides;

h) - la sélection parmi ces bactéries transformées de celles qui ont inséré un fragment d'ADN humain, de manière à obtenir une banque de clones;

i) - l'isolement de cette banque des clones porteurs de l'ADN-AT par crible caractéristique de l'$\alpha$1-antitrypsine humaine.

j) - l'identification parmi ces clones de ceux qui expriment l'$\alpha$1-antitrypsine humaine.

La présente invention concerne également des clones d'Escherichia Coli porteurs de l'ADN-AT et produisant l'$\alpha$1-antitrypsine humaine. L'invention concerne en plus l'ADN complémentaire double brin codant pour l'$\alpha$1-antitrypsine complète désigné par ADN-AT, qui a été isolé des clones et des vecteurs qui le portent.

a) Les ARN messagers totaux de foie humain peuvent être extraits par différentes méthodes basées essentiellement sur la destruction complète des structures cellulaires et la dénaturation rapide et totale des activités ribonucléasiques dans l'extrait. On trouvera dans "Molecular Cloning" (1982 - Maniatis, T., Fritsch, E.F. and Sambrook, J.-C.S.H. Publ.) l'état de l'art en cette matière.

A titre d'exemple, on peut recourir à l'emploi combiné de détergents, tels le NP40, et d'inhibiteurs de ribonucléase, tels le vanadyl-ribonucléoside, ou encore à l'emploi d'agents chaotropiques puissants, tels les sels de guanidine.

De préférence, on choisit la méthode décrite dans Cox, R.A. (Methods Enzymol 12B, 120, 1968), basée sur l'utilisation de chlorure de guanidine, qui conduit à l'obtention d'acides ribonucléiques non dégradés et actifs.

Les ARN messagers totaux extraits de foie humain peuvent être purifiés en tirant parti d'une de leurs caractéristiques, à savoir la présence d'une séquence polyadénylique à leur extrémité 3'. La méthode de choix consiste à réaliser une chromatographie d'affinité sur colonne d'oligo d(T) cellulose, selon le protocole décrit dans Avis, H. and Leder, P. (1972, Proc. Natl. Acad. Sci. USA, 69, 1408).

b) L'enrichissement des ARN messagers totaux en molécules codant pour l'$\alpha$ 1-antitrypsine humaine repose sur des techniques de fractionnement, telles l'électrophorèse sur gel d'agarose et la centrifugation en gradient de saccharose. De préférence, on choisit le fractionnement sur gradient de saccharose tel qu'il est décrit dans Rougeon, F., Chambraud, B., Foote, S., Panthier, J.J., Nageotte, R. and Corvol, P. (1981) Proc. Natl. Acad. Sci. USA, 78, 6367.

c) La conversion enzymatique in vitro des ARN messagers polyadénylés en ADN complémentaire double brin fait appel à des réactions enzymatiques dont la description détaillée est reprise dans Genetic Engineering (1981) vol. 1, pp. 9-19, Williamson, R., Ed., Academic Press, N.Y. A titre d'exemple, on utilise successivement les enzymes appelés, transcriptase inverse, ADN polymérase et nucléase S1 pour synthétiser l' ADN complémentaire double brin portant des extrémités franches. On peut cependant utiliser uniquement la transcriptase inverse et la nucléase S1 pour arriver au même résultat.

Enfin, on peut terminer la préparation de l'ADN complémentaire double brin portant des extrémités franches par un traitement à l'ADN polymérase (fragment
Klenow) pour pallier aux imperfections du traitement
avec la nucléase S1. De préférence, on choisit cette
dernière voie dans l'exécution de l'invention.

d) On peut enrichir la préparation d'ADN complémentaire
double brin en molécules de taille correspondant à
l'ADN-AT en recourant aux techniques de fractionnement rapportées au paragraphe 2. De préférence,
dans la réalisation de l'invention, on choisit le
fractionnement sur gradient de saccharose.

e) Différentes méthodes ont été utilisées pour joindre.
l'ADN complémentaire double brin à l'ADN de vecteurs
plasmides (Molecular Cloning, Maniatis, T., Fritsch
E.F. and Sambrook, J., 1982, C.S.H. Publ.). Les
plus courantes consistent à ajouter des extensions
homopolymériques complémentaires aux ADN que l'on
veut joindre, ou des adapteurs synthétiques à l'ADN
que l'on veut introduire dans un site de restriction
particulier de l'ADN du vecteur. De préférence, on
choisit l'addition d'extrémités cohésives complémentaires aux ADN que l'on veut joindre. Cette technique, décrite dans Villa- Komaroff, L., Efstradiatis,
A., Broome, S., Lomedico, P., Tizard, R., Naker,
S.P., Chick, W.L. and Gilbert, W. (1978, Proc. Natl.
Acad. Sci. USA. 75, 3727) met en oeuvre une enzyme
particulière appelée terminal deoxynucléotidyl
transférase.

f) La formation in vitro de molécules hybrides entre
l'ADN complémentaire double brin à cloner et l'ADN
de vecteurs plasmides s'effectue selon le procédé,
décrit dans Molecular Cloning (1982 - Maniatis, T.,
Fritsch, E.F. and Sambrook, J., C.S.H. Publ.) qui
consiste à mélanger les molécules dans des conditions
de température et de force ionique appropriées.

C'est ce protocole qui a été suivi dans la réalisation de l'invention. L'ADN vecteur employé dans la réaction de recirculation in vitro peut provenir de différents plasmides. Parmi les plasmides, les plus couramment utilisés, on rencontre le plasmide pBR322 qui contient deux sites d'insertion possible, PstI et BamHI. Ces deux sites conviennent à l'insertion de l'ADN susmentionné. (Bolivar, F., Rodriguez, R.L., Greene, P.J., Betlach, M.C., Heynecker, H.L., Boyer, H.W., Crosa, J.H. and Falkow, S., Gene, 2,95, 1977). Les dérivés du plasmide pBR322 peuvent aussi convenir. Dans la réalisation de cette invention, on choisit de préférence le plasmide pBR322 dont on linéarise l'ADN et que l'on munit d'extrémités cohésives appropriées selon la méthode décrite plus haut. D'autres plasmides, tels que le pK 279, pK 280 et pK 287 peuvent être utilisés avantageusement.

g) La plupart des méthodes utilisées pour transformer des bactéries par de l'ADN exogène font appel à un traitement particulier des bactéries par du chlorure de calcium, et visent à optimiser l'efficacité de transformation pour différentes souches de bactéries. Dans le cadre de l'invention, plusieurs types de bactéries peuvent servir d'hôtes aux vecteurs utilisés. On prendra de préférence, la souche d'Escherichia Coli MM294 dont les conditions de croissance sont particulièrement commodes.

En outre, parmi les différents procédés de transformation, on choisit celui décrit dans Mandel, M. and Higa, A. (1970, J. Mol. Biol. 53, 154).

h) En raison des marqueurs de résistance aux antibiotiques présents dans l'ADN du vecteur, il est simple d'isoler les bactéries transformées par l'ADN recombiné in vitro. Par exemple, si l'ADN du vecteur porte le gène Tet$^R$, toute bactérie portant un tel vecteur sera résistante à la tetracycline.

En outre, si l'insertion de l'ADN dans le vecteur a pour effet d'inactiver un gène de résistance à un antibiotique, on repérera les bactéries qui portent le vecteur recombinant par leur sensibilité à cet antibiotique. De préférence, on choisit comme marqueur de sélection, le caractère Ter$^R$ du plasmide pBR322 et comme crible pour l'insertion d'ADN dans le vecteur la perte de résistance à l'ampicilline. La combinaison de ces deux facteurs conduit à l'identification de bactéries portant un vecteur recombinant.

i) Le crible de sélection des clones porteurs de l'ADN-AT peut se faire selon des méthodes mettant en oeuvre soit une sonde naturelle ou synthétique, soit des techniques d'hybridation entre l'ADN des clones et l'ARN messager, ou encore des moyens immunologiques. Ces différentes approches sont détaillées dans Genetic Engineering, vol. 1, Williamson, R. Eds., (1981) Academic Press, N.Y.

Dans le cadre de la présente invention, on utilise de préférence une sonde oligodésoxyribonucléotique de synthèse, caractéristique des acides aminés Gly Ala Met Phe Leu de l'$\alpha$ 1-antitrypsine et/ou le procédé de rétention de l'ARN messager spécifique de l'$\alpha$ 1-antitrypsine humaine sur l'ADN des clones, suivi de la traduction in vitro et de la caractérisation immunologique.

j) L'identification de clones qui expriment l'$\alpha$ 1-antitrypsine humaine repose d'une part sur la construction de vecteurs recombinants dans lesquels l'ADN-AT est inséré dans la bonne orientation et dans la phase correcte de lecture, et d'autre part sur la caractérisation immunologique du produit synthétisé dans les bactéries portant ces vecteurs hybrides.

Les procédés relatifs à ces constructions sont détaillés dans Molecular Cloning (1982 - Maniatis, T., Fritsch, E.F. and Sambrook, J., C.S.H. Publ.). Si besoin est, le vecteur utilisé, qu'il s'agisse du vecteur pBR322 ou de tout autre vecteur peut être modifié pour assurer la lecture en phase correcte de l'ADN-AT qui y est inséré. Ceci peut être réalisé notamment par la production de vecteurs portant des délétions dans le gène où se fait l'insertion de l'ADN, conduisant ainsi à un décalage de une, deux ou trois bases entre le point d'initiation de la transcription et la première base de l'ADN-AT. Ce type de vecteurs permettant les trois phases de lecture est décrit dans Talmadge, K, Stahl, S. and Gilbert, W., Proc. Natl. Acad. Sci. USA, 77, 3369 (1980).

Après insertion de l'ADN-AT dans chacun de ces vecteurs, on comprend que seul l'un d'eux sera en tout état de cause apte à être traduit correctement par les bactéries hôtes appropriées avec, pour conséquence, la production d'une protéine hybride contenant celle qui correspond à l'ADN-AT.

Dans le cadre de l'invention, on choisit de préférence les vecteurs pBR322 et pK279, pK280, pK287 pour la mise en phase correcte et un procédé de détection immunologique basé sur l'emploi d'anticorps fixé sur matrice. L'exemple non limitatif suivant qui s'applique au clonage de l'ADN-AT dans le vecteur plasmide pBR322 permettra de mieux comprendre l'invention.

1. Extraction et purification des ARN messagers totaux de foie humain.

10 g de foie humain prélevé par autopsie sur un individu sain ont été traités au chlorure de guanidine suivant la méthode de Cox, R.A., Methods Enzymol. 12B, 120, 1968. Ceux-ci sont alors passés sur colonne d'oligo d(T) cellulose de façon à en récupérer les ARN messagers totaux.

Au départ de 10 g de foie humain, on a obtenu 200µg d'ARN messagers totaux. La fraction messagère ainsi obtenue est alors traduite en polypeptides in vitro dans un système protéo-synthétique acellulaire issu de réticulocytes de lapin (produit notamment par NEN Laboratories). L'ensemble des protéines synthétisées est alors fractionné sur gel de polyacrylamide (15%) en conditions dénaturantes. On peut s'assurer de la présence d'$\alpha$1-antitrypsine parmi les produits synthétisés in vitro en immunoprécipitant spécifiquement celle-ci par un anticorps dressé contre l'$\alpha$1-antitrypsine (produit commercial Nordic) et en analysant l'immunoprécipité par électrophorèse. Ces expériences confirment bien la présence d'$\alpha$1-antitrypsine dans les produits de traduction (Mw=48000 daltons) et donc celle du messager correspondant dans la fraction messagère totale.

2. Enrichissement de la préparation d'ARN messagers totaux en molécules codant pour l'$\alpha$1-antitrypsine

On s'est attaché ensuite à isoler une fraction messagère enrichie en ARN messagers codant pour l'$\alpha$1-antitrypsine, en fait, enrichie en messager codant pour des protéines de Mw voisin de 50000 daltons. A cet effet, 60 µg d'ARN messagers totaux ont été fractionnés, selon la taille, sur gradient de saccharose de concentration de 10 à 30 %. Après centrifugation en rotor Beckman SW41, 30 fractions de 400 µl ont été récupérées et testées in vitro pour la synthèse d'$\alpha$1-antitrypsine en combinant l'analyse immunologique et l'électrophorèse sur les produits de traduction. Ces expériences ont permis d'identifier une fraction messagère dans laquelle l'abondance de messager codant pour l'$\alpha$1-antitrypsine était importante. Cette fraction correspondant à un ARN messager d'environ 14 à 15 S contenait 6 µg de matériel.

3. Synthèse in vitro de l'ADN complémentaire double brin correspondant à la fraction messagère enrichie.

Au départ de l'ARN messager enrichi pour l'$\alpha$1-antitrypsine, on a synthétisé l'ADN qui lui correspond. Cette opération requiert la succession de plusieurs étapes enzymatiques. D'une part, à l'aide de la transcriptase réverse, on obtient la fibre d'ADN complémentaire au messager, et d'autre part, à l'aide de la même enzyme ou de l'ADN polymérase, on synthétise la deuxième fibre d'ADN antiparallèle à la première. Enfin, l'action d'une troisième enzyme, la nucléase S1, permet l'obtention de molécules d'ADN en double hélice dépourvues d'extrémités monocaténaires appelées usuellement extrémités franches. L'ensemble des réactions ci-dessus a mis en oeuvre 6 μg de RNA messager enrichi pour l'$\alpha$1-antitrypsine et a conduit à l'obtention de 520 ng d'ADN complémentaire double brin.

4. Enrichissement de la préparation d'ADN complémentaire double brin en molécules de taille correspondant à l'ADN-AT.

La population de molécules d'ADN complémentaire étant hétérogène, on l'a fractionnée sur gradient de saccharose de façon à ne conserver que la fraction contenant des molécules de taille supérieure à 1,1 kilobases, et donc à favoriser l'obtention de clones contenant la majorité, sinon l'entièreté, de l'information génétique correspondant à l'$\alpha$1-antitrypsine. La taille théorique du messager de l'$\alpha$1-antitrypsine codant pour ses 394 acides aminés comporte 394 codons, soit 1182 bases.

A cet effet, 520 ng d'ADN complémentaire double brin ont été déposés sur un gradient de saccharose 10-30% et centrifugés en rotor Beckman SW41.

Les fractions correspondant à des molécules de taille plus grande ou égale à 1,1 kilo-bases ont été rassemblées; elles contiennent 100 ng d-ADN complémentaire double brin.

5. **Addition d'extrémités cohésives à la préparation d'ADN complémentaire double brin enrichie en ADN-AT.** Afin de pouvoir insérer les molécules d'ADN complémentaire double brin dans l'ADN d'un vecteur approprié, il convient de leur ajouter des extrémités cohésives. A cet effet, on adopte la technique des extensions homopolymériques décrite par Villa-Komaroff, L., Efstradiatis, A., Broome, S., Lomedico, P., Tizard, R., Naker, S.P. Chick, W.L. and Gilbert, W. (Proc. Natl. Acad. Sci. U.S.A. 75, 3727, 1978). Dans ce protocole, 100 ng d'ADN complémentaire double brin enrichi ont été traités par la transférase terminale de façon à ajouter aux extrémités 3' des extensions d'oligo d(C) d'environ 15 bases de long.

6. **Fabrication in vitro de recombinants entre l'ADN complémentaire double brin enrichi, portant des extrémités cohésives, et l'ADN du vecteur pBR322.** Les molécules d'ADN complémentaire double brin enrichi sont maintenant prêtes à être insérées dans l'ADN d'un vecteur plasmide. On a choisi le plasmide pBR322, portant les marqueurs de sélection Tet$^R$ et Amp$^R$ (résistance à la tétracycline et à l'ampicilline). L'ADN de ce plasmide porte un site unique pour l'enzyme de restriction PstI, dans le gène AmpA. Dès lors, la restriction par PstI linéarise le plasmide et offre un site d'insertion unique. Il suffit alors d'ajouter enzymatiquement par la méthode décrite en 5. des extensions oligo d(G) aux extrémités 5' de l'ADN du vecteur pour pouvoir réaliser l'insertion de l'ADN à cloner. Dans l'exemple que l'on décrit, l'ADN du vecteur pBR322 porte des extensions oligo d(G) longues de 30 bases.

Le mélange de 70 ng d'ADN complémentaire double brin enrichi avec 200 ng d'ADN de vecteur pBR322, ce qui correspond à un rapport molaire voisin de 1, s'effectue in vitro pendant 5 min. à 65°C et 1 h 30 à 57° C. Le mélange est alors refroidi doucement jusqu'à 4°C. Dans ces conditions les molécules d'ADN se recombinent et se recircularisent par appariement des extrémités cohésives complémentaires.

7. Clonage dans la souche d'Escherichia coli MM294.

On procède alors à la transformation de la souche MM294 dont le système de restriction-modification est modifié de façon à tolérer la présence d'un ADN étranger selon la méthode décrite dans Molecular Cloning, Maniatis, T., Fritsch; E.F. and Sambrook, J., 1982, C.S.H. publ.

Au sein de la bactérie, les extrémités cohésives incomplètes sont réparées par une enzyme de l'hôte avec pour conséquence la régénération du site de restriction PstI dans l'ADN du plasmide recombinant.

8. Obtention de la banque de clones.

En raison du caractère Ter$^R$ du plasmide, on peut sélectionner les bactéries transformées par simple croissance sur milieu contenant de la tétracycline. Enfin, parmi les souches Ter$^R$, on peut identifier celles qui ont un plasmide chimérique par le fait qu'elles ne poussent pas en présence d'ampicilline puisque l'insertion d'ADN étranger dans le gêne AmpA du vecteur a inactivé ce gène.

L'ensemble des manipulations décrites ci-dessus a conduit à l'obtention d'une banque de 5000 clones, dont une grande partie doit contenir des séquences d'ADN codant pour l'α1-antitrypsine.

9. <u>Caractérisation des clones porteurs de l'ADN-AT.</u>

On a recherché les clones α1-antitrypsine, d'une part, au moyen d'une sonde synthétique correspondant à un fragment du gène α1-antitrypsine et, d'autre part, en combinant l'hybridation de l'ADN des clones avec le messager spécifique de l'α1-antitrypsine suivi de la traduction in vitro et de la caractérisation immunologique.

<u>Synthèse et utilisation d'une sonde synthétique.</u>

La séquence en nucléotides d'un fragment de l'ADN codant pour l'antitrypsine humaine est connue. Sur base de cette information, on synthétise chimiquement selon la méthode de Hsiung, H.M., Brosseau, R., Michnicwicz, J. and Narang, S.A., 1979, Nucleic Acid Research 6, 1371 un oligodésoxyribonucléotide de 12 bases correspondant aux acides aminés Gly-Ala-Met-Phe-Leu, typiques de l'α1-antitrypsine humaine (positions 349 à 353 dans la protéine). Après avoir marqué cette sonde au $^{32}$P par une réaction enzymatique (kination en 5' de la sonde),nous l'avons utilisée pour cribler la banque de clones. Pratiquement, on fixe l'ADN de chaque clone sur feuille de nitrocellulose, suivant la technique de Gruustein, M. and Hogness, D., Proc. Natl. Acad. Sci. USA 72, 3961, 1975 et on l'hybride avec la sonde synthétique marquée au $^{32}$P Dans des conditions ioniques et de température appropriées (0,9 M NaCl et 28°C), la sonde radioactive va reconnaître spécifiquement l'ADN des clones portant une séquence homologue, c'est-à-dire une séquence de nucléotides typique de l'α1-antitrypsine humaine. On visualise les clones positifs par autoradiographie. Cette méthode a permis d'identifier 25 clones (sur 500 analysés) portant tout ou partie de l'ADN-AT.

<u>Rétention du messager spécifique de l'α 1-antitrypsine</u>
<u>sur l'ADN des clones.</u>

Indépendamment du crible de la banque de clones par la
sonde synthétique, on recherche les clones α 1-antitryp-
sine par la méthode suivante : l'ADN plasmidique de
clones, groupés par quatre, est extrait, linéarisé par
une enzyme de restriction (HindIII), puis fixé sur
disque de nitrocellulose. Ces disques sont alors mis
en contact avec l'ARN messager total extrait de foie
humain dans des conditions permettant l'hybridation
spécifique des ARN messagers correspondant aux ADN
fixés sur les disques suivant la méthode de Ricciardi,
R.P., Miller, J.S. and Roberts, B.E., 1979, Proc. Natl.
Acad. Sci.USA 76, 4927. On peut alors récupérer les
ARN messagers hybridés les traduire in vitro dans un
système de réticulocytes de lapin, analyser les produits
de traduction tels quels ou après immunoprécipitation
avec un anticorps dressé contre l' α 1-antitrypsine.
Cette méthode a conduit à l'identification de groupes
de clones contenant des séquences codantes pour l'α 1-
antitrypsine. On peut ensuite refaire le même type de
manipulations sur chaque clone isolé et identifier sans
ambiguité celui qui contient l'information génétique de
l'α 1-antitrypsine. Ces expériences ont conduit à l'identification de 4 clones portant l'ADN-AT sur 32 clones
analysés.

<u>Recherche d'autres clones porteurs de l'ADN-AT</u>

On isole le fragment d'ADN inséré dans un clone positif
pour les deux cribles mentionnés ci-dessus. Cet ADN a
été marqué au $^{32}P$ par la méthode dite du déplacement
de coupure (nick translation) décrite par Kelly, R.G.,
Cozzarelli, N., Deutscher, M.P., Lehman, I.R.and
Kornberg, A. (1970, Methods Enzymol. 73, 442), puis
utilisé comme sonde d'hybridation avec l'ADN des clones
de la banque fixé sur nitrocellulose.

L'hybridation s'est effectuée à 65°C dans des conditions ioniques appropriées (0.9 M NaCl). Dans ces conditions, la sonde radioactive a révélé 95 clones contenant des séquences d'ADN-AT sur les 500 clones analysés. Pour une partie de ces clones positifs, on mesure la taille du fragment d'ADN-AT cloné. Ceci s'effectue par l'isolement de l'ADN plasmidique de chaque clone, la restriction de cet ADN par l'enzyme PstI et l'analyse sur gel d'agarose des fragments d'ADN. Etant donné que le poids moléculaire de l'$\alpha$ 1-antitrypsine non glycosylée est de $\pm$ 48000 daltons (ou $\pm$ 400 acides aminés), on doit s'attendre à trouver des clones dont l'insert est proche de $\pm$ 1200 paires de bases, si l'entièreté de la séquence codante a été clonée.

Les résultats montrent que, parmi les différents clones analysés, plusieurs portent des inserts de taille compatible avec la totalité de l'information génétique de l'$\alpha$ 1-antitrypsine. Nous avons choisi de caractériser un clone, appelé ci-après pULB1523, qui porte un fragment d'ADN-AT d'environ 1370 paires de bases.

Caractérisation de l'ADN-AT cloné dans le clone pULB1523 On caractérise l'ADN-AT du clone pULB1523 par l'établissement d'une carte de restriction. A cet effet, l'ADN inséré, isolé du plasmide par restriction PstI, est digéré par une ou plusieurs autres enzymes de restriction. L'alignement des fragments obtenus permet d'obtenir une carte de restriction. On peut comparer cette carte à celle que l'on connaît déjà, d'une part pour l'ADN d'$\alpha$ 1-antitrypsine de babouin et, d'autre part, pour un fragment 5' et un fragment 3' de l'ADN codant pour l'$\alpha$ 1-antitrypsine humaine. On s'aperçoit que certains sites de restriction, et aussi que la distance entre certains sites particuliers, TaqI-AvaI et AvaI-HinfI, etc..., se retrouve dans notre clone comme on s'y attend sur base des sites présents dans la fraction 3' de l'ADN $\alpha$ 1-antitrypsine humaine.

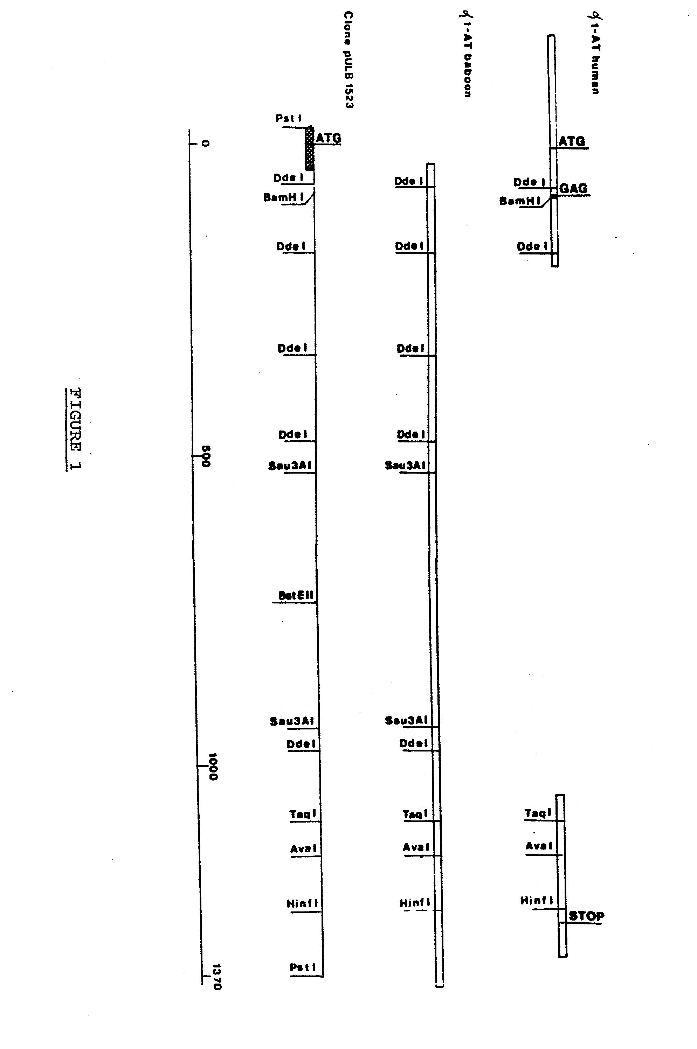

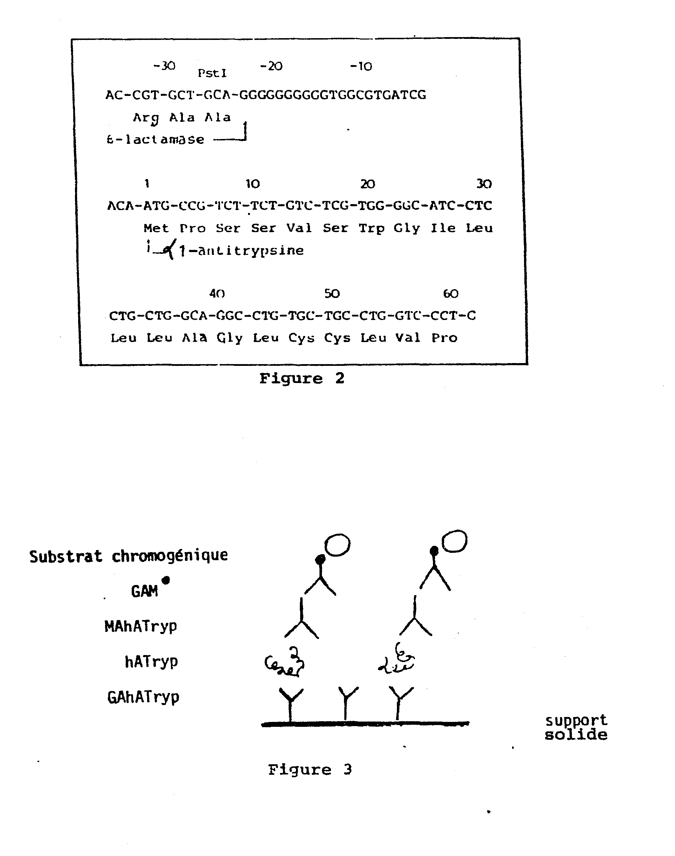

Ces informations permettent d'orienter le fragment d'ADN cloné, de situer le codon fin de chaîne, le début de la protéine mûre (codon GAG), le début du précurseur (codon ATG), et d'estimer la longueur de la protéine codée par cet ADN. Etant donné notamment que le site BamHI (position 105) est présent dans l'insert, il s'avère que l'on a un ADN qui code pour au moins 360 acides aminés de l'$\alpha$ 1-antitrypsine, soit quasiment la totalité de la protéine. La figure 1 donne la carte de restrictions de l'ADN-AT inséré dans le clone pULB 1523. Afin de s'assurer que l'ADN-AT cloné dans pULB1523 code pour l'entièreté de l'$\alpha$ 1-antitrypsine humaine, on a déterminé la séquence en nucléotides de la région 5' de l'ADN cloné. Cette expérience repose sur la disponibilité d'un site de restriction BamHI (position 105) permettant de générer des fragments d'ADN que l'on peut aisément marquer au $^{32}$P. Le fragment marqué est alors soumis à une série de réactions chimiques conduisant à la production d'oligonucléotides marqués, de tailles différentes, dont l'analyse sur gel de polyacrylamide conduit à l'établissement de la séquence en bases, suivant la technique de Maxam, A.M. and Gilbert, W., 1977, Proc. Natl. Acad. Sci. USA 74, 560. On voit dans la figure 2, que l'ADN cloné dans pULB1523 contient le codon initiateur ATG et que la séquence des bases en aval de ce triplet correspond à la séquence connue en acides aminés du peptide signal de l'$\alpha$ 1-antitrypsine. On peut donc en conclure que le clone pULB1523 code pour la totalité de la protéine humaine $\alpha$ 1-antitrypsine.

10. **Expression de l'ADN-AT dans la bactérie Escherichia Coli MM294.**

Le fragment d'ADN-AT porté par le clone pULB1523 non seulement code pour l'entièreté de l'$\alpha$ 1-anti-trypsine, mais est aussi inséré dans le sens correct de transcription par rapport au promoteur du gène AmpA et dans la phase correcte de lecture. Ces conclusions découlent à la fois de la carte de restriction du clone pULB1523 reprise à la fig.1 et de la séquence en bases de la région initiatrice dans l'insert reprise à la fig. 2. On doit s'attendre, dès lors, à pouvoir mettre en évidence l'expression de l'ADN-AT cloné, c'est-à-dire la synthèse par la bactérie MM294 d'une protéine portant des déterminants antigéniques typiques de l'$\alpha$ 1-antitrypsine humaine. Par ailleurs, on a construit une série de trois vecteurs hybrides portant l'ADN codant pour l'$\alpha$ 1-antitrypsine dans l'orientation correcte et dans chacune des trois phases de lecture. Dans ce cas, on s'attend à observer la synthèse de la protéine humaine uniquement dans la bactérie portant le vecteur hybride ayant l'insert dans la phase de lecture correcte. La construction de ces trois vecteurs hybrides met en oeuvre les étapes suivantes :

- isolement de l'ADN-AT par restriction PstI de l'ADN du vecteur hybride pULB1523;

- mélange de cet ADN-AT avec l'ADN des trois vecteurs pK279, pK280 et pK287 linéarisés par restriction PstI

- ligation des fragments d'ADN par une enzyme (ligase) pendant 16 heures à 4°C;

- on procède alors à la transformation de la bactérie MM294 et à la sélection des bactéries trans-formées.

0120829

18

Parmi celles-ci, on identifie celles qui portent l'ADN-AT dans l'orientation correcte en réalisant une carte de restriction des trois vecteurs hybrides. On obtient ainsi trois souches, appelées pK279-3, pK280-24 et pK287-31 caractérisées par le fait que chacune d'entre elles porte l'ADN-AT dans la bonne orientation et dans une des trois phases de lecture.

Pour démontrer la synthèse d'$\alpha$1-antitrypsine humaine dans les bactéries MM294 portant les vecteurs hybrides pULB1523, pK279-3, pK280-24 et pK287-31, on s'appuie sur un essai immunologique permettant de détecter la protéine dans des extraits bactériens totaux. Le principe de l'expérimentation est illustré dans la figure 3 où les abréviations utilisées ont la signification suivante :

GAM$^\bullet$     immunoglobulines de chèvre dressées contre les immunoglobulines de souris et marquées à la peroxydase.

MahATryp     anticorps de souris dressés contre l'$\alpha$1-antitrypsine humaine.

hATryp     $\alpha$1-antitrypsine humaine témoin ou présente dans les extraits bactériens.

GAhATryp     anticorps de chèvre dressés contre l'$\alpha$1-antitrypsine humaine, fixés sur support solide.

Le substrat chromogénique(O)réagit avec la peroxydase couplée au GAM, conduisant à une coloration orange que l'on mesure à 495 nmètres.

Lorsqu'on applique ce système aux extraits provenant des bactéries portant les différents vecteurs, on constate que, comme on s'y attend, la souche témoin portant le plasmide pBR322 ne synthétise pas d'$\alpha$1-antitrypsine, alors que, pour le clone pULB1523, on détecte la présence de cette protéine.

En outre, parmi les souches portant chacun des trois vecteurs hybrides, seule celle, qui porte le vecteur où l'ADN-AT est dans la phase correcte de lecture, synthétise de l' $\alpha$1-antitrypsine en quantié relativement importante.

Revendications

1. Procédé de préparation d'un clone bactérien produisant l'α 1 antitrypsine humaine, caractérisé en ce que on extrait et purifie les ARN messagers totaux de foie humain, on enrichit ces messagers en molécules codant pour l'α 1 - antitrypsine humaine, on effectue la synthèse in vitro de l'A D.N. complémentaire double brin au départ de cette fraction messagère enrichie, on enrichit la préparation d'ADN complémentaire double brin en molécules de taille correspondant à celle de l'A D N - A T., on ajoute des extrémités cohésives à cette préparation d'A D N complémentaire double brin enrichie en A D N - A T, on mélange in vitro cette fraction enrichie portant des extrémités cohésives avec un vecteur plasmide portant des extrémités cohésives complémentaires pour obtenir des molécules hybrides, on transforme des bactéries hôtes Eschérichia Coli par ces molécules hybrides, on sélectione parmi ces bactéries transformées celles qui ont inséré un fragment d'A D N humain de manière à obtenir une banque de clones, on isole de cette banque les clones porteurs de l'A D N - A T par crible caractéristique de l'α 1 - antitrypsine.humaine, on identifie parmi ces clones ceux qui expriment l'α 1-antitrypsine humaine.

2. Procédé de préparation d'un clone bactérien produisant l'α 1 - antitrypsine humaine suivant la revendication 1, caractérisé en ce que le vecteur plasmide est choisi parmi le pBR322, pK279, pK280 et pK287.

3. Procédé de préparation d'un clone bactérien produisant l'α 1 - antitrypsine humaine suivant les revendications 1 et 2, caractérisé en ce que l'A D N. complémentaire enrichi en A D N - A T est inséré dans le site PstI du gène AmpA du vecteur plasmide.

4. Procédé de préparation d'un clone bactérien produisant l'α 1 - antitrypsine humaine suivant les revendications 1 à 3, caractérisé par l'utilisation comme crible d'une sonde oligodésoxyribonucléo-tidique synthétique caractéristique des acides aminé Gly-Ala-Met-Phe-Leu de l'α 1 - antitrypsine et/ou du procédé basé sur la rétention du messager spécifique de l'α 1 - antitrypsine humaine sur l'A D N du clone.

5. Procédé de préparation d'un clone bactérien produisant l'$\alpha$1-anti-trypsine humaine, caractérisé par l'extraction des A R N messagers totaux de foie humain par utilisation de chlorure de guanidine et purification par chromatographie d'affinité sur colonne d'oligo.d(T) cellulose, l'enrichissement des messagers par fractionnement sur gradient de saccharose, la synthèse in vitro de l'A D N complémentaire double brin par utilisation de la transcriptase inverse et de la nucléase S1 et par traitement ultérieur avec l'A D N polymérase, l'enrichissement de la préparation d'A D N complémentaire double brin en molécules de taille correspondant à celle de l'A D N - A T par fractionnement sur gradient de saccharose, l'addition des extrémités cohésives à la préparation précédente par utilisation de l'enzyme appelée transférase deoxynucléotidyle terminale, le mélange in vitro de cette fraction enrichie portant des extrémités cohésives avec un vecteur plasmide du type pBR322, la transformation d'Escherichia Coli MM294 par ces molécules hybrides, la sélection des bactéries qui ont inséré un fragment d'A D N humain en choisissant comme marqueur de sélection le caractère Tet$^R$ du plasmide pBR322 et comme crible pour l'insertion d'A D N dans le vecteur la perte de résistance à l'ampicilline, l'utilisation comme crible d'une sonde oligo-désoxyribonucléotique synthétique et/ou du procédé de rétention de l'ARN messager spécifique de l'$\alpha$1-antitrypsine humaine sur l'A D N des clones, l'identification des clones qui expriment l'$\alpha$1-antitrypsine humaine par la construction de vecteurs recombinant dans lequels l'A D N - A T est inséré dans la bonne orientation et dans la phase correcte de lecture et par la caractérisation immunologique du produit synthétisé dans les bactéries porteurs des vecteurs hybrides par l'emploi d'anticorps fixé sur matrice.

6. A D N complémentaire double brin codant pour le polypeptide $\alpha$1-antitrypsine complet désigné par A D N - A T obtenu suivant les revendications 1 à 4.

7. Clones d'Escherichia Coli portant l'A D N - A T et produisant l'$\alpha$1-antitrypsine humaine obtenu suivant les revendications 1 à 5.

α1-AT human

α1-AT baboon

Clone pULB 1523

FIGURE 1

```
        -30      PstI    -20           -10
AC-CGT-GCT-GCA-GGGGGGGGGGGTGGCGTGATCG
     Arg Ala Ala
ß-lactamase ──┘


     1            10            20          30
ACA-ATG-CCG-TCT-TCT-GTC-TCG-TGG-GGC-ATC-CTC
    Met Pro Ser Ser Val Ser Trp Gly Ile Leu
      ⊥∢1-antitrypsine

          40           50          60
CTG-CTG-GCA-GGC-CTG-TGC-TGC-CTG-GTC-CCT-C
Leu Leu Ala Gly Leu Cys Cys Leu Val Pro
```

Figure 2

Substrat chromogénique

GAM•

MAhATryp

hATryp

GAhATryp

support solide

Figure 3